Europäisches Patentamt

European Patent Office

Office européen des brevets

(11) Numéro de publication : **0 368 770 B1**

(19)

(11)

## (12) FASCICULE DE BREVET EUROPEEN

(45) Date de publication du fascicule du brevet :
**14.10.92 Bulletin 92/42**

(51) Int. Cl.$^5$ : **C07C 205/22, C07C 201/08**

(21) Numéro de dépôt : **89420427.0**

(22) Date de dépôt : **06.11.89**

(54) **Procédé de préparation de nitrophenols.**

(30) Priorité : **07.11.88 FR 8815185**

(43) Date de publication de la demande :
**16.05.90 Bulletin 90/20**

(45) Mention de la délivrance du brevet :
**14.10.92 Bulletin 92/42**

(84) Etats contractants désignés :
**AT BE CH DE ES FR GB GR IT LI LU NL SE**

(56) Documents cités :
**EP-A- 0 211 775**
**FR-A- 2 144 395**
**GB-A- 1 098 717**

(72) Inventeur : **Baudouin, Michel Val Fontaine**
**35, rue des Grottes Saint Genis Les Ollières**
**F-69290 Craponne (FR)**
Inventeur : **Bougeois, Jean-Luc**
**4, chemin du Signal**
**F-69110 Sainte-Foy Les Lyon (FR)**
Inventeur : **Lecouve, Jean-Pierre**
**23 E, rue de l'Oratoire**
**F-69300 Caluire (FR)**
Inventeur : **Ratton, Serge**
**Hameau de Belmont Vaulx Milieu**
**F-38090 Villefontaine (FR)**

(74) Mandataire : **Dutruc-Rosset, Marie-Claude et al**
**RHONE-POULENC CHIMIE Direction des Brevets 25, Quai Paul Doumer**
**F-92408 Courbevoie Cédex (FR)**

(73) Titulaire : **RHONE-POULENC CHIMIE**
**25, quai Paul Doumer**
**F-92408 Courbevoie Cédex (FR)**

## Description

La présente invention concerne un procédé continu de préparation de nitrophénols par nitration du phénol en milieu aqueux.

La nitration du phénol est une réaction connue depuis longtemps et les publications sur ce sujet sont innombrables et très souvent divergentes en ce qui concerne le mécanisme de la réaction, ou plus exactement des réactions en compétition (nitration, nitrosation oxydation, dinitration, polycondensation). Un des problèmes essentiels auquel on se heurte lorsqu'on veut nitrer le phénol est celui de la sélectivité de la nitration. En effet, la nitration directe du phénol conduit à un rapport orthonitrophénol/paranitrophénol qui est sensiblement de 50/50. Or les débouchés industriels de l'orthonitrophénol sont beaucoup moins importants que ceux du paranitrophénol. Il en résulte que, pour satisfaire les besoins en paranitrophénol, il est préparé également de l'orthonitrophénol en quantités très exédentaires par rapport à la demande.

Différentes solutions ont été proposées pour favoriser l'obtention du dérivé para, voire pour n'obtenir que du dérivé para. Il a été proposé de préparer tout d'abord le paranitrosophénol par nitrosation à l'aide de l'acide nitreux, puis d'oxyder le paranitrosophénol en paranitrophénol. Un tel procédé est économiquement peu rentable. En outre, le nitrosophénol est un composé instable qui présente des risques d'explosion thermique et qu'il est donc préférable de ne pas accumuler en grosses quantités.

Il a été décrit dans le brevet GB 1 098 717 la nitration du phénol par l'acide nitrique en milieu aqueux, en présence d'acide nitreux et d'acide sulfurique, avec des concentrations déterminées de ces différents acides, notamment de fortes concentrations en acide sulfurique. Selon les variantes de ce procédé , il y a soit une précipitation de solide dans le réacteur, soit, pour éviter une telle précipitation, un débit très lent d'introduction du phénol. Sur le plan industriel, un tel procédé pose d'importants problèmes techniques (forte concentration d'acide sulfurique, précipitation d'un solide, faible productivité).

Pour remédier à ces inconvénients, le brevet français publié sous le numéro 2 144 395, décrit un procédé consistant à faire réagir le phénol ou l'un de ses dérivés, dans un mélange aqueux d'acide nitrique et d'acide nitreux, à une température de -15°C à + 15°C, en maintenant une certaine concentration d'acide nitrique et un rapport molaire global acide nitrique/phénol de 4/1 à 5/1.

Ce procédé conduit à un mélange réactionnel pâteux, que l'on réchauffe en fin d'addition du phénol afin de dissoudre les solides ou suspension. C'est donc en fait un procédé en 2 étapes, qu'il est difficile de réaliser industriellement en continu.

La présente invention se propose de permettre de réaliser en continu la nitration directe du phénol en nitrophénols avec un rapport paranitrophénol/orthonitrophénol supérieur ou égal à 55/45.

Plus précisément l'invention consiste en un procédé continu de préparation de nitrophénols en milieu aqueux, caractérisé en ce qu'en régime :

- on introduit en continu du phénol et de l'acide nitrique à raison d'environ 0,5 à 2,0 moles d'acide nitrique par mole de phénol, dans un réacteur fermé, agité, contenant un mélange réactionnel constitué essentiellement par une solution aqueuse comprenant de 5 % à 40 % en poids d'acide nitrique, de l'acide nitreux à raison de 5 % à 40 % en poids par rapport au poids d'acide nitrique et des nitrophénols dans la limite de leur solubilité dans le milieu,
- la température dans le réacteur est maintenue entre 10°C et 40°C environ,
- et on soutire en continu un mélange constitué essentiellement de paranitrophénol et d'orthonitrophénol avec un rapport isomérique paranitrophénol/orthonitrophénol supérieur ou égal à 55/45.

De préférence la concentration de la solution aqueuse du mélange réactionnel en acide nitrique sera de 10 % à 25 % en poids par poids.

Le rapport pondéral acide nitreux/acide nitrique dans la couche aqueuse du mélange réactionnel sera de préférence de 10% à 20 %. La température du mélange réactionnel sera de préférence maintenue entre 15°C et 30°C environ.

Le phénol est introduit généralement sous forme liquide. Il peut donc, soit être introduit à l'état fondu, soit être introduit en mélange avec de l'eau. Dans ce dernier cas des mélanges comportant de 70 à 90 % en poids de phénol conviennent bien. Bien que l'on puisse utiliser des mélanges eau-phénol contenant moins de 70 % en poids de phénol, cela n'est guère intéressant au plan industriel, car il faudra éviter l'accumulation de cette eau et donc prévoir un système d'extraction de l'excès d'eau du milieu réactionnel.

L'acide nitrique peut également être introduit à l'état pur ou sous la forme d'une solution aqueuse.

Pour la même raison qu'indiquée précédemment, l'introduction de l'acide nitrique pur permet d'éviter d'introduire de l'eau. Par contre pour des raisons économiques, il peut être préférable d'utiliser des solutions aqueuses commerciales d'acide nitrique telles que par exemple la solution à environ 68 % d'acide nitrique, qui correspond au mélange azéotropique eau/acide nitrique.

Le mélange de nitrophénols formé décante sous la forme d'un liquide huileux à la partie inférieure, non soumise à l'agitation, du réacteur ou dans un 2ème appareil servant de décanteur et relié à la partie inférieure du réacteur.

Ce mélange constitué essentiellement des mononitrophénols contient également de l'eau, un peu d'acide nitrique dissous, ainsi que des sous-produits de la réaction tels que des dinitrophénols et de la benzoquinone.

Si la quantité d'eau ainsi éliminée est inférieure à la quantité d'eau, d'une part formée par la réaction de nitration et d'autre part éventuellement portée par le phénol et l'acide nitrique introduits, il est nécessaire d'éliminer périodiquement cet excès d'eau.

Pour cela, on peut par exemple prélever, quand on l'estime nécessaire, une partie de la couche aqueuse présente dans le réacteur.

Ce prélèvement de couche aqueuse peut être concentré pour en retirer la quantité voulue d'eau, puis être réintroduit dans le réacteur.

Il peut aussi être effectué de telle façon que cela corresponde à la quantité d'eau qu'il est nécessaire d'enlever et, dans ce cas, il n'y a pas de recyclage de ce prélèvement, la perte en acide nitrique étant alors compensée en agissant sur la quantité d'acide nitrique introduite en continu.

Le rapport molaire, de l'acide l'acide nitrique et du phénol introduits en continu dans le procédé de l'invention, sera ajusté en fonction du taux de transformation souhaité du phénol et du recyclage ou du non-recyclage des prélèvements de la couche aqueuse du mélange réactionnel, après traitement et/ou concentration desdits prélèvements. Lorsque ceux-ci sont recyclés, la perte en acide nitrique est moins importante et un rapport molaire acide nitrique/phénol de 0,9 à 1,5 convient généralement bien.

La pression dans le réacteur est constituée par la pression autogène des différents réactifs et produits formés.

Elle est généralement maintenue entre 0,1 MPa (1 bar) et 1 MPa (10 bars) absolu.

Bien que ce ne soit pas le cas généralement, il n'est pas exclu de réaliser le procédé selon l'invention, en maintenant dans le réacteur une pression absolue inférieure à 0,1 MPa.

En raison de la formation de sous-produits gazeux, il est nécessaire d'ajuster la pression dans le réacteur à la valeur choisie soit périodiquement, soit de manière permanente par l'intermédiaire d'un système de dégazage fonctionnant dès que la valeur choisie de pression dans le réacteur est atteinte.

Les gaz formant le ciel du réacteur, et dont on élimine ainsi une partie, sont constitués essentiellement par du dioxyde de carbone et par différents oxydes d'azote dont le plus important en quantité est NO.

De préférence la pression autogène dans le réacteur est maintenue entre 0,1 MPa et 0,5 MPa absolu.

Le procédé selon l'invention fonctionnant en continu et générant des sous-produits, ceux-ci sont essentiellement soutirés avec les nitrophénols. Cependant, certains peuvent être partiellement solubles dans la phase aqueuse qui demeure dans le réacteur. Il peut donc y avoir progressivement une certaine accumulation de tels sous-produits dans le réacteur.

Il est aisé, pour le bon fonctionnement du procédé, de prévoir périodiquement une purge de la couche aqueuse contenant les acides nitriques et nitreux. Cette purge peut consister en un prélèvement de la couche aqueuse, et en un traitement de ce prélèvement consistant à oxyder les sous-produits en dioxyde de carbone et eau à l'aide de l'acide nitrique présent. Cela peut être effectué par exemple en chauffant le prélèvement dans un appareil annexe, puis, après élimination des sous-produits, en réintroduisant la solution traitée dans le réacteur.

Il est bien évident que l'on peut combiner l'élimination de l'excès éventuel d'eau du mélange réactionnel avec l'élimination des sous-produits, par exemple en chauffant préalablement la partie prélevée de la couche aqueuse pour oxyder les sous-produits, puis en distillant l'excès d'eau avant de réintroduire dans le réacteur le prélèvement épuré et concentré.

Le procédé selon l'invention possède de nombreux avantages. Il n'y a pratiquement pas de nitrosophénol décelable dans le réacteur de nitration, contrairement aux procédés de nitrosation-oxydation de l'art antérieur. Les risques liés à l'accumulation de ce composé sont donc supprimés.

La réaction s'effectue en une étape et non en deux étapes : nitrosation suivie d'une oxydation du nitrosophénol en nitrophénol. Le milieu réactionnel est liquide et est constitué essentiellement par la phase aqueuse dans laquelle s'effectue la réaction de nitration et le mélange des nitrophénols produits qui décante à la partie inférieure du réacteur ou dans un décanteur et qui est soutirée.

Le procédé est également très souple, puisqu'il suffit par exemple d'agir sur la température dans le réacteur pour obtenir, selon les besoins, un rapport paranitrophénol/orthonitrophénol plus au moins élevé. En règle générale l'augmentation de la température diminue le rapport paranitrophénol/orthonitrophénol, toutes choses étant égales par ailleurs.

Enfin, contrairement aux procédés antérieurs, il n'y a pas nécessité d'ajout d'acide nitreux ou de l'un de ses précurseurs (tel que NO ou $NO_2$) lorsque le procédé est en régime : seuls le phénol et l'acide nitrique sont alimentés en continu. L'acide nitreux n'a de fait qu'un rôle catalytique.

La mise en régime du procédé selon l'invention peut se faire de différentes manières.

Il est possible d'introduire dans le réacteur une solution aqueuse d'acide nitrique présentant une concentration de 5 à 40 % en poids, puis, après avoir fixé une température de 10°C à 40°C, commencer la nitration en introduisant en continu le phénol et l'acide nitrique avec un rapport molaire phénol/acide titrique de 0,5 à 2.

Pendant une certaine période que l'on appellera période d'induction, le rapport isomérique paranitrophénol/orthonitrophénol du mélange de nitrophénols soutiré, sera sensiblement de 50/50. Après cette période d'induction, de quelques minutes à quelques heures environ, le procédé sera en régime et le rapport paranitrophénol/orthonitrophénol sera supérieur ou égal à 55/45, selon notamment la température adoptée.

Cette période d'induction peut également être totalement supprimée en introduisant au début de la mise en oeuvre du procédé et uniquement à ce moment, de l'acide nitreux ou l'un des ses précurseurs dans le réacteur, c'est à dire soit de l'acide nitreux, soit un nitrite alcalin tel que nitrite de sodium, soit une pression de 0,1 MPa à 0,5 MPa de NO, soit à la fois de l'acide nitreux et l'un des ses précurseurs.

L'appareillage dans lequel peut être réalisé le procédé de l'invention est constitué essentiellement :
- par un réacteur muni des moyens d'agitation, et de chauffage et de refroidissement, et à partie inférieure duquel peut être soutirée une partie de son contenu ;
- par des moyens d'introduction des réactifs : phénol et acide nitrique ;
- par des moyens de dégazage et de régulation de la pression ;
- par des moyens de prélèvement d'une partie de la couche aqueuse pour purge et de réintroduction, le cas échéant, dudit prélèvement après traitement approprié.

Le réacteur peut être constitué de 2 éléments :
- Le réacteur proprement dit où s'effectue la réaction et dont le niveau est maintenu constant,
- un décanteur dans lequel sont séparées en continu : la phase aqueuse réactionnelle qui est recyclée en permanence et la phase nitrophénols qui est soutirée.

Le traitement du mélange de nitrophénols se fait par tout moyen habituel tel que distillation, entraînement à la vapeur, recristallisation.

## EXEMPLE 1

### Description de l'appareillage

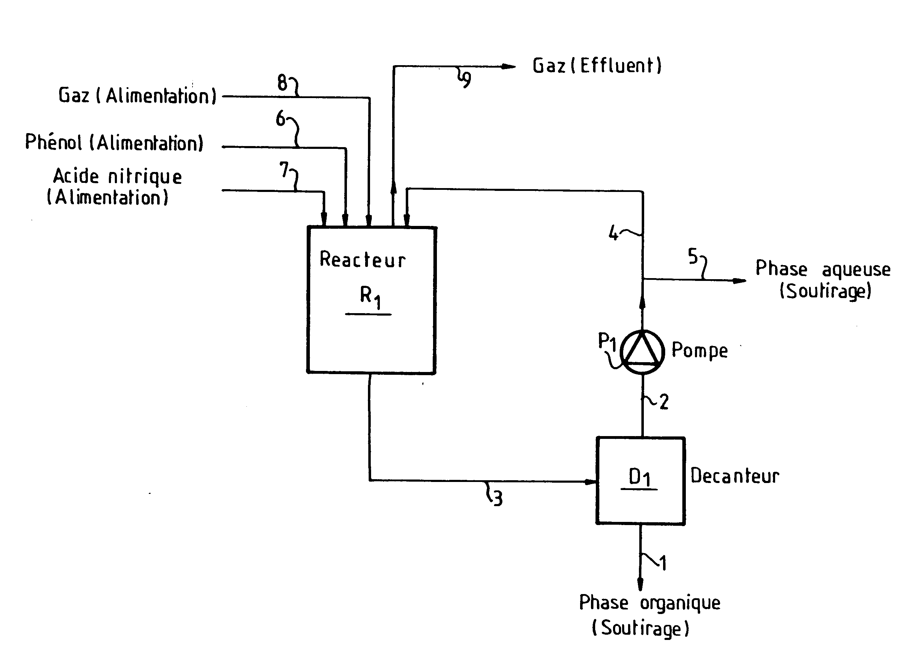

L'appareillage utilisé est constitué (voir schéma de la planche unique)
- d'un réacteur $R_1$ cylindrique en verre d'une capacité utile de 1 litre, agité au moyen d'une turbine ; cet appareil peut être refroidi par circulation d'eau dans une double enveloppe ;
- d'un décanteur $D_1$ constitué d'un cylindre horizontal en verre d'une capacité de 0,16 litre avec une sortie inférieure (1) et une sortie supérieure (2) ;
- d'un dispositif de pompe $P_1$ et de tuyauteries permettant d'alimenter (3) en continu le décanteur par le contenu liquide du réacteur et de renvoyer (4) après décantation la couche liquide supérieure du décanteur vers le réacteur ou (5) vers un dispositif de purge.

Le réacteur est également équipé de dispositifs permettant d'alimenter en continu une solution aqueuse de phénol (6) d'une part et une solution aqueuse d'acide nitrique (7) d'autre part. Il comporte enfin un dispositif permettant d'introduire (8) et de laisser dégager (9) des gaz à pression atmosphérique.

### conduite de la réaction en continu

### Charges initiales

Le réacteur $R_1$ et le décanteur $D_1$ sont chargés par 901,5 g d'une solution aqueuse contenant 2,810 moles d'acide nitrique et 0,379 mole d'acide nitreux. Ce mélange est préparé initialement par absorption de l'oxyde d'azote NO dans une solution aqueuse diluée d'acide nitrique.

Le mélange est agité et maintenu à une température de 25°C et circule continuellement du décanteur $D_1$ vers le réacteur $R_1$, au moyen de la pompe $P_1$ avec un débit de 7 litres/heure.

### Conduite en continu de la réaction

Dans le réacteur $R_1$ on alimente en continu :
- 117,1 g/heure d'un mélange $HNO_3$ - Eau à 68 % de $HNO_3$ ;
- 114,8 g/heure d'un mélange phénol-eau à 80,4 % de phénol.

Le contenu du réacteur est maintenu à 25°C par circulation d'eau froide dans la double-enveloppe.

Après 35 minutes de fonctionnement le mélange réactionnel se sépare en deux phases liquides :

- une phase organique inférieure constituée principalement de nitrophénols,

- une phase aqueuse supérieure contenant $HNO_3$, $HNO_2$, ainsi que des nitrophénols à saturation.

La phase organique décantée est soutirée à la base du décanteur $D_1$ (1) alors que la phase aqueuse est renvoyée par $P_1$ dans le réacteur $R_1$ (4).

Le contenu de l'ensemble du réacteur et du décanteur est maintenu constant en volume de liquide, par soutirage en continu d'une fraction de la phase aqueuse au refoulement de la pompe $P_1$ (5).

Les gaz produits par la réaction, principalement constitués de $CO_2$ et de NO, sont évacués par simple dégagement à pression atmosphérique (9).

La composition de la phase organique et de la phase aqueuse soutirées en continu est déterminée par analyses en chromatographie en phase liquide et également par potentiométrie.

Des prélèvements instantanés de la phase aqueuse effectués toutes les 3 heures, sont clarifiés par centrifugation et analysés. Le tableau n° 1 montre l'évolution de la composition au cours du temps.

La phase organique soutirée au décanteur $D_1$ (1), collectée toutes les 3 heures, est pesée et analysée. La masse et la composition de chaque fraction est représentée dans le tableau n° 2.

Durant la totalité de l'expérience (12 heures) la phase aqueuse décantée et soutirée de l'installation représente une masse de 978,6 g. Un dégazement constant (9) d'une quantité totale de 39,4 litres de gaz (mesuré à 25°C et à pression atmosphérique) a été observé à la sortie du réacteur $R_1$. Ce gaz contient 49 % en volume d'oxyde d'azote NO.

Le bilan global de l'expérience après 12 h de marche montre qu'il a été engagé 11,77 moles de phénol et qu'il a été récupéré :

- 0,084 mole de phénol (Taux de transformation 99,3 %)
- 6,886 mole de paranitrophénol (RT* = 58,9 %)
- 3,525 mole d'orthonitrophénol (RT* = 30,2 %)

*RT : rendement par rapport au phénol transformé.

TABLEAU n° 1 - COMPOSITION DE LA PHASE AQUEUSE

| Durée en heures<br>Concentration | 0 | 3 | 6 | 9 | 12 |
|---|---|---|---|---|---|
| HNO 3<br>mole/kg | 3,117 | 2,433 | 2,419 | 2,317 | 2,346 |
| HNO 2<br>mole/kg | 0,420 | 0,276 | 0,281 | 0,312 | 0,264 |
| p.nitrophénol<br>mole/kg | 0 | 0,298 | 0,260 | 0,257 | 0,250 |
| O.nitrophénol<br>mole/kg | 0 | 0,071 | 0,040 | 0,037 | 0,036 |

TABLEAU n° 2 - PHASE ORGANIQUE SOUTIREE

| Fraction | 1 | 2 | 3 | 4 |
|---|---|---|---|---|
| Période de production | 0 à 3H | 3H à 6H | 6H à 9H | 9H à 12H |
| Masse (g.) | 285,7 | 421 | 439 | 441 |
| P-nitrophénol % en poids | 51,4 | 52,4 | 53,5 | 56,7 |
| O-nitrophénol % en poids | 34,0 | 26,6 | 27,4 | 26,2 |
| phénol % en poids | non déterminé | 0,16 | 0,17 | 0,17 |
| Benzoquinone % en poids | 1,07 | 0,67 | 1,16 | 0,96 |
| Dinitrophénols % en poids | 1,1 | 0,57 | 0,39 | 0,32 |
| Eau % en poids | 11,1 | 11,8 | 11,8 | 11,8 |

## EXEMPLE 2

L'expérience est réalisée dans l'appareillage décrit dans l'exemple n° 1.

### Charge initiale

Le réacteur $R_1$ et le décanteur $D_1$ sont chargés par 915 g d'une solution aqueuse contenant 2,956 moles d'acide nitrique. (Pas d'acide nitreux).

### Réaction

### Phase d'induction

Le mélange est agité et maintenu à une température de 28°C. Il circule continuellement du décanteur $D_1$ vers le réacteur $R_1$ au moyen de la pompe $P_1$ avec un débit de 8 litres/heure.

On ajoute en continu à débit constant au réacteur $R_1$, 69 grammes d'un mélange phénol-eau à 80,5 % de

7

phénol ; la durée d'introduction est de 44 min, la température du mélange contenu dans le réacteur $R_1$ est maintenu à 28°C par circulation d'eau froide dans la double enveloppe.

L'addition de phénol est alors interrompue durant 37 min et la température de 28°C est maintenue durant cette période.

Poursuite en continu de la réaction

A partir de la phase d'induction décrite précédemment, on alimente en continu dans le réacteur $R_1$ :
- 118,5 g/h d'un mélange $HNO_3$-eau à 68 % de $NHO_3$.
- 118,5 g/h d'un mélange phénol-eau à 80,5 % de phénol.

La phase organique décantée est soutirée en continu à la base du décanteur $D_1$ (1) alors que la phase aqueuse est renvoyée par $P_1$ dans le réacteur $R_1$ (4).

Le contenu de l'ensemble du réacteur et du décanteur et maintenu constant en volume de liquide par soutirage en continu d'une fraction de la phase aqueuse au refoulement de la pompe $P_1$ (5).

Les gaz produits par la réaction sont évacués du réacteur de manière à maintenir une pression interne constante égale à la pression atmosphérique (9).

Durant une première période de 45 minutes la température du mélange réactionnel est progressivement amenée de 28°C à 25°C, puis est maintenue à cette dernière valeur durant encore 7h 15 min.

Des prélèvements instantanés effectués à différents moments de la phase aqueuse et de la phase organique sont analysés (tableaux n° 3 et 4)

Un bilan matière effectuée durant 4h 11 min de marche continue de la réaction montre qu'il a été engagé 4,252 moles de phénol et qu'il a été récupéré :
- 0,015 mole de phénol (Taux de transformation 99,6 %)
- 2,503 moles de paranitrophénol (RT* = 59,1 %)
- 1,163 mole d'orthonitrophénol (RT* = 27,5 %)

*RT = rendement par rapport au phénol transformé.

8

TABLEAU n° 3 - COMPOSITION DE LA PHASE AQUEUSE
-------------------------------------------------

| Durée en heures *  Concentration | 1 | 2 | 4 | 6 | 8 |
|---|---|---|---|---|---|
| HNO 3 mole/kg | 2,417 | 2,113 | 2,171 | 2,211 | 2,230 |
| HNO 2 mole/kg | 0,114 | 0,201 | 0,196 | 0,223 | 0,205 |
| p.nitrophénol mole/kg | 0,207 | 0,144 | 0,229 | 0,232 | 0,234 |
| O.nitrophénol mole/kg | 0,036 | 0,023 | 0,033 | 0,034 | 0,036 |

* A partir du début de la conduite en continu de la réaction.

EP 0 368 770 B1

TABLEAU n° 4 – COMPOSITION DE LA PHASE ORGANIQUE

| Durée en heures *  Concentration | 1 | 2 | 4 | 6 | 8 |
|---|---|---|---|---|---|
| p.nitrophénol % en poids | 50,8 | 52,3 | 52,7 | 52,7 | 53,5 |
| o.nitrophénol % en poids | 31,5 | 26,6 | 24,9 | 24,9 | 25,3 |
| phénol % en poids | 0,09 | 0,12 | 0,11 | 0,06 | 0,22 |
| benzoquinone % en poids | 2,5 | 2,1 | 1,8 | 1,8 | 1,9 |
| dinitrophénols % en poids | 0,21 | 0,15 | 0,15 | 0,14 | 0,15 |

* A partir du début de la conduite en continu de la réaction.

## Revendications

1) Procédé continu de préparation de nitrophénols en milieu aqueux, caractérisé en ce qu'en régime :
- on introduit en continu du phénol et de l'acide nitrique à raison d'environ 0,5 à 2,0 moles d'acide nitrique par mole de phénol, dans un réacteur fermé, agité, contenant un mélange réactionnel constitué essentiellement par une solution aqueuse comprenant de 5 % à 40 % en poids d'acide nitrique, de l'acide nitreux à raison de 5 % à 40 % en poids par rapport au poids d'acide nitrique et des nitrophénols dans la limite de leur solubilité dans le milieu,
- la température dans le réacteur est maintenu entre 10°C et 40°C environ,
- et on soutire en continu un mélange constitué essentiellement de paranitrophénol et d'orthonitrophénol avec un rapport isomérique paranitrophénol/orthonitrophénol supérieur ou égal à 55/45
2) Procédé selon la revendication 1, caractérisé en ce que la pression autogène dans le réacteur est de 0,1 MPa à 1 MPa et de préférence de 0,1 MPa à 0,5 MPa.

3) Procédé selon l'une des revendications 1 ou 2, caractérisé en ce que la concentration en acide nitrique de la couche aqueuse du mélange réactionnel est de 10 % à 25 % en poids.

4) Procédé selon l'une des revendications 1 à 3, caractérisé en ce que le rapport pondéral acide nitreux/acide nitrique dans la couche aqueuse du mélange réactionnel est de 10 % à 20 %.

5) Procédé selon l'une des revendications 1 à 4, caractérisé en ce que la température du mélange réactionnel est maintenue entre 15°C et 30°C environ.

6) Procédé selon l'une des revendications 1 à 5, caractérisé en ce que le rapport molaire entre l'acide nitrique et le phénol introduit est de 0,9 à 1,5.

## Patentansprüche

1. Kontinuierliches Verfahren zur Herstellung von Nitrophenolen in wäßrigem Medium, dadurch gekennzeichnet, daß man während des Betriebs:
   - kontinuierlich Phenol und Salpetersäure in einem Verhältnis von etwa 0,5 bis 2,0 mol Salpetersäure je mol Phenol in einen geschlossenen Rührreaktor einführt, der ein Reaktionsgemisch enthält, das im wesentlichen aus einer wäßrigen Lösung besteht, die 5 bis 40 Gew.-% Salpetersäure, salpetrige Säure in einem Verhältnis von 5 bis 40 Gew.-%, bezogen auf das Gewicht der Salpetersäure, und Nitrophenole in der Grenze ihrer Löslichkeit in dem Medium umfaßt,
   - die Temperatur in dem Reaktor in einem Bereich von 10°C bis etwa 40°C hält,
   - und kontinuierlich ein Gemisch abzieht, das im wesentlichen aus p-Nitrophenol und o-Nitrophenol mit einem Verhältnis der Isomeren p-Nitrophenol/o-Nitrophenol größer oder gleich 55/45 besteht.

2. Verfahren nach Anspruch 1, dadurch gekennzeichnet, daß der autogene Druck im Reaktor 0,1 MPa bis 1 MPa, vorzugsweise 0,1 MPa bis 0,5 MPa beträgt.

3. Verfahren nach einem der Ansprüche 1 oder 2, dadurch gekennzeichnet, daß die Konzentration an Salpetersäure der wäßrigen Schicht des Reaktionsgemisches 10 bis 25 Gew.-% beträgt.

4. Verfahren nach einem der Ansprüche 1 bis 3, dadurch gekennzeichnet, daß das Gewichtsverhältnis von salpetriger Säure zu Salpetersäure in der wäßrigen Schicht des Reaktionsgemisches 10 bis 20 % beträgt.

5. Verfahren nach einem der Ansprüche 1 bis 4, dadurch gekennzeichnet, daß man die Temperatur des Reaktionsgemisches bei 15 bis etwa 30°C hält.

6. Verfahren nach einem der Ansprüche 1 bis 5, dadurch gekennzeichnet, daß das Molverhältnis zwischen eingebrachter Salpetersäure und eingebrachtem Phenol 0,9 bis 1,5 beträgt.

## Claims

1. Continuous process for the preparation of nitrophenols in aqueous medium, characterized in that, in a steady state:
   - phenol and nitric acid are introduced continuously at a rate of approximately 0.5 to 2.0 moles of nitric acid per mole of phenol into a closed stirred reactor containing a reaction mixture consisting essentially of an aqueous solution comprising from 5% to 40% by weight of nitric acid, nitrous acid in a proportion of 5% to 40% by weight relative to the weight of nitric acid and nitrophenols within the limit of their solubility in the mixture,
   - the temperature in the reactor is maintained between 10°C and approximately 40°C,
   - and a mixture consisting essentially of para-nitrophenol and ortho-nitrophenol with a para-nitrophenol/ortho-nitrophenol isomer ratio higher than or equal to 55/45 is withdrawn continuously.

2. Process according to claim 1, characterized in that the autogenous pressure in the reactor is from 0.1 MPa to 1 MPa and preferably from 0.1 MPa to 0.5 MPa.

3. Process according to either of claims 1 and 2, characterized in that the concentration of nitric acid in the aqueous layer of the reaction mixture is from 10 % to 25 % by weight.

4. Process according to one of claims 1 to 3, characterized in that the nitrous acid/nitric acid weight ratio in the aqueous layer of the reaction mixture is from 10 % to 20 %.

5. Process according to one of claims 1 to 4, characterized in that the temperature of the reaction mixture is maintained between 15°C and approximately 30°C.

6. Process according to one of claims 1 to 5, characterized in that the molar ratio of nitric acid to the phenol which is introduced is from 0.9 to 1.5.

Gaz (Effluent)

Gaz (Alimentation) — 8

Phénol (Alimentation) — 6

Acide nitrique
(Alimentation) — 7

Reacteur

R₁

Phase aqueuse
(Soutirage)

P₁ Pompe

Decanteur

D₁

Phase organique
(Soutirage)

EP 0 368 770 B1